# EUROPEAN PATENT APPLICATION

(11) **EP 3 135 773 A1**
(43) Date of publication of application: **01.03.2017**
(21) Application number: 16180991.8
(22) Date of filing: 25.09.2007
(51) Int. Cl.: C12Q 1/68

(54) **METHODS FOR BREAST CANCER PROGNOSIS**

(30) Priority: 27.09.2006 EP 06020209
(62) Divisional of application: 07820546.5
(71) Applicant: Sividon Diagnostics GmbH, 50829 Cologne (DE)
(72) Inventor: Gehrmann, Mathias, 51375 Leverkusen (DE); von Törne, Christian, 42659 Solingen (DE)
(74) Representative: dompatent von Kreisler Selting Werner - Partnerschaft von Patent- und Rechtsanwälten mbB

(57) **Abstract**

The present invention relates to methods, kits and systems for the prognosis of the disease outcome of breast cancer in untreated breast cancer patients. More specific, the present invention relates to the prognosis of breast cancer based on measurements of the expression levels of marker genes in tumor samples of breast cancer patients. Marker genes are disclosed which allow for an accurate prognosis of breast cancer in patients having node negative, fast proliferating breast cancer.

## Description

### Technical Field

The present invention relates to methods, kits and systems for the prognosis of the disease outcome of breast cancer in untreated breast cancer patients. More specific, the present invention relates to the prognosis of breast cancer based on measurements of the expression levels of marker genes in tumor samples of breast cancer patients. Marker genes are disclosed which allow for an accurate prognosis of breast cancer in patients having node negative, fast proliferating breast cancer.

### Background of the Invention

Expression of estrogen receptor alpha and proliferative activity of the breast tumors have long been recognized to be of prognostic importance. Patients with ER positive tumors tend to have a better prognosis than ER negative patients (Osborne et al, 1980) and rapid proliferating tumors tend to have a worse outcome (Gentili et al, 1981). Knowledge about the molecular mechanisms involved in the processes of estrogen dependent tumor growth and proliferative activity has led to the successful development of therapeutic approaches, i.e. anti-endocrine and cytotoxic chemotherapy.

Gene expression profiling has greatly extended the possibility to analyze the underlying biology of the heterogeneous nature of breast cancer. Perou and co-worker (2000) described breast cancer subtypes identified after two dimensional hierarchical clustering which they referred to as luminal, basal-like, normal-like and ERBB2-like breast cancer subtypes. These subtypes differed in their clinical outcome and response to chemotherapy (Sorlie et al, 2001; Sorlie et al, 2003; Rouzier et al, 2005). However, the list of genes used to define these subtypes changed often and proliferation genes were largely neglected in the early publications. Furthermore, a simple, reproducible and comprehensible classification algorithm was not deduced. In a more statistically driven case control design, also called supervised analysis, two different groups identified genes differentially expressed in tumors of node negative and untreated patients who developed a metastasis within five years or remained disease free for at least five years van't Veer et al, 2002; Wang et al, 2005). The respective classification algorithms outperformed all other conventional prognostic factors and were confirmed in subsequent validation studies (van de Vijver et al, 2002; Foekens et al, 2006). However, since both lists overlapped by only 3 genes considerable uncertainty about the validity and general applicability of these findings arose in the medical community (Brenton et al, 2005). Meanwhile it is becoming increasingly clear, that most prognostic and predictive classification algorithms rely predominantly on the measurement of estrogen receptor alpha regulated genes and genes involved in the cell cycle (Paik et al, 2004; Sortiriou et al, 2006; Oh et al, 2006).

Another potential prognostic factor, which was largely unattended in gene expression studies, is the immune system. Tumor infiltration by lymphocytes has long been suggested to influence clinical outcome (Aaltomaa et al, 1992).

In particular, medullary breast cancer (MBC), which is characterized by prominent lymphocytic infiltrates, is linked with relatively good outcome despite estrogen receptor negativity and poor histological grade (Ridolfi et al, 1977). Recently, MBC has been identified to be closely related to basal like tumors (Bertucci et al, 2006) which suggests that the poor outcome of the basal subtype could be improved by the influence of the immune system.

Several groups showed that luminal / ER positive breast cancer has a significantly better outcome than basal / ER negative breast cancer (Sorlie et al, 2001; 2003; Chang et al, 2005). The importance of ER status in breast cancer was further underlined by the finding that ER positive and ER negative tumors display remarkably different gene expression phenotypes not solely explained by differences in estrogen responsiveness (Gruvberger et al, 2001). A reciprocal relationship in the expression levels of genes responsible for prediction of ER status and S-Phase of the cell cycle as a marker for proliferation has been suggested (Gruvberger-Saal et al, 2004). These two factors, ER and proliferation, are major determinants of breast cancer biology. Indeed, several recent studies have focused on the association between proliferation and ER in predicting survival in breast cancer (Perreard et al, 2006; Dai et al, 2005).

A relationship between host defense mechanisms and prognosis of breast cancer has been discussed for decades (Di Paola et al, 1974). However, conflicting results led to dispute about the actual role of tumor-associated leucocytes (O'Sullivan and Lewis, 1994). Nonetheless, lymphocytic infiltrates were related to good outcome in breast cancer, especially in rapidly proliferating tumors (Aaltomaa et al, 1992). Menard and co-worker (1997) showed in a comprehensive study of 1919 breast carcinomas an independent prognostic influence of lymphoid infiltration only in younger patients. Since younger patients commonly have more rapidly proliferating tumors as compared to older patients, we focused on the subgroup of tumors with high expression of the proliferation metagene.

Immunophenotyping of tumor-infiltrating lymphocytes (TIL) reveals a preponderance of T cells as compared to B cells (Chin et al, 1992; Gaffey et al, 1993). T cells have an important role both in innate, non-specific immunity and in adaptive, antigen-specific immunity. Given the frequency of tumor-infiltrating T cells as compared with B cells, earlier studies analyzed preferentially the significance of tumor-infiltrating T cells in breast cancer. However, these studies yielded inconsistent results regarding the prognostic significance of T cells (Shimokawara et al, 1982; Lucin et al, 1994).

More recently, several reports focused on oligoclonal expansion of B cells both in MBC (Coronella et al, 2001, Hansen et al, 2001) and in ductal breast carcinoma (DBC) (Coronella et al, 2002; Nzula et al, 2003). Hansen and co-workers (2002) described an oligoclonal B cell response targeting actin which was exposed on the cell surface as an early apoptotic event in MBC. The observed IgG antibody response showed all criteria of an antigen-driven, high-affinity response. Furthermore, ganglioside D3 was identified as another target for an oligoclonal B cell response in MBC (Kotlan et al, 2005). These authors interpreted their findings as proof of principle concerning tumor-infiltrating B lymphocytes. Despite tempting implications regarding the prognostic impact of these findings, none of these studies actually analyzed the significance of the described B cell response for survival.

US 2004/0229297-A1, filed 27 January 2004, discloses a method for the prognosis of the breast cancer in a patient said method comprising detecting in human tumor tissues the infiltration of certain immune cells. High infiltration of the tumor with immune cells was associated with poor cancer prognosis. The method, however, does not use information on the nodal status and does not rely on information on the rate of proliferation of the tumor.

In regard to the continuing need for materials and methods useful in making clinical decisions on adjuvant therapy, the present invention fulfills the need for advanced methods for the prognosis of breast cancer on the basis of readily accessible clinical and experimental data.

### Summary of the Invention

The present invention is based on the surprising finding that the outcome of breast cancer in breast cancer patients, not receiving chemotherapy, can be accurately predicted from the expression levels of a small number of marker genes in node-negative patients, having fast proliferating tumors. It has been found that the expression of said marker genes are most informative, in this specific group of patients. As the proliferation status of a tumor can also be assessed from gene expression experiments, the present method allows to collect all necessary data from a single gene chip experiment. Accordingly, the present invention relates to prognostic methods for the determination of the outcome of breast cancer in non-treated breast cancer patients, using information on the nodal status of the patient, on the expression of marker genes being indicative of the proliferation status of the tumor, and information on the expression level of a second marker gene, predictive for the outcome of the disease in said patient. The second marker genes are preferably specifically expressed in immune cells, such as T-cells, B-cells or natural killer cells.

### Detailed description of the Invention

The present invention relates to a method for the prognosis of breast cancer in a breast cancer patient, said method comprising
(a) determining the nodal status of said patient;
(b) determining the expression level of at least one first marker gene in a tumor sample from said patient, said first marker gene providing information on whether said tumor is fast proliferating or slow proliferating;
(c) determining whether said tumor is a fast proliferating tumor or a slow proliferating tumor, by comparison of said expression level of said first marker gene with a predetermined first threshold level;
(d) determining the expression level of at least one second marker gene in a tumor sample of said patient, wherein it is preferred that said second marker gene is specifically expressed in immune cells;
wherein a favorable prognosis is given, if said nodal status is negative and said tumor is a fast proliferating tumor and said expression level of said second marker gene is above a predetermined threshold level, and
wherein an unfavorable prognosis is given if said nodal status is negative and said tumor is a fast proliferating tumor and said expression level of said second marker gene is below a predetermined threshold level.

"Prognosis", within the meaning of the invention, shall be understood to be the prediction of the outcome of a disease under conditions where no systemic chemotherapy is applied in the adjuvant setting.

The present invention further relates to methods for the prognosis of breast cancer in a breast cancer patient in which said prognosis is based on the information that said nodal status is negative and on information on the that said tumor is a fast proliferating tumor and on information on the said expression level of said second marker gene.

For a prognostic method to "be based" on a multiple pieces of information (as is the case in the present invention) all individual pieces of information must be taken into consideration for arriving at the prognosis. This means that all individual pieces of information can influence the outcome of the prognosis. It is well understood that a piece of information, such as e.g. the nodal status of a patient, can influence the outcome of the prognosis in that the prognostic method is only applied when said nodal status is e.g. negative. Likewise, it is understood that a method can "be based" on information relating to the proliferation rate of the tumor, e.g. if fast proliferation is a conditional criterion applied in the course of the prognostic method.

In preferred methods of the invention, said prognosis is entirely based on the information that said nodal status is negative and that said tumor is a fast proliferating tumor and on information on the expression level of said second marker gene in said tumor sample.

In preferred methods of the invention, said prognosis is an estimation of the likelihood of metastasis fee survival of said patient over a predetermined period of time, e.g. over a period of 5 years.

In further preferred methods of the invention, said prognosis is an estimation of the likelihood of death of disease of said patient over a predetermined period of time, e.g. over a period of 5 years.

"Death of disease", within the meaning of the invention, shall be understood to be the death of a breast cancer patient after recurrence of the disease.

"Recurrence", within the meaning of the invention, shall be understood to be the recurrence of breast cancer in form of metastatic spread of tumor cells, local recurrence, contralateral recurrence or recurrence of breast cancer at any site of the body of the patient.

In specific embodiments of the invention, the breast cancer patient is not treated with cancer chemotherapy in the adjuvant setting.

In preferred methods of the invention, the expression of said first marker gene is indicative of fast proliferation of the tumor.

In preferred methods of the invention, said first marker gene is selected from Table 1.

In specific embodiments of the invention, a single, or 2, 5, 10, 20, 50 or 100 first marker genes are used.

**Table 1:**

| **Probe Set** | **Classification** | **Gene Symbol** | **Location** |
|---|---|---|---|
| 222039_at | Proliferation | LOC146909 | Chr:17q21.31 |
| 218662_s_at | Proliferation | HCAP-G | Chr:4p16-p15 |
| 221520_s_at | Proliferation | FLJ10468 | Chr:1p34.2 |
| 218755_at | Proliferation | KIF20A | Chr:5q31 |
| 204825_at | Proliferation | MELK | Chr:9p13.1 |
| 218542_at | Proliferation | C10orf3 | Chr:10q23.33 |
| 204444_at | Proliferation | KIF11 | Chr:10q24.1 |
| 218039_at | Proliferation | ANKT | Chr:15q14 |
| 202705_at | Proliferation | CCNB2 | Chr:15q21.2 |
| 218009_s_at | Proliferation | PRC1 | Chr:15q26.1 |
| 210052_s_at | Proliferation | C20orf1 | Chr:20q11.2 |
| 202954_at | Proliferation | UBE2C | Chr:20q13.11 |
| 202095_s_at | Proliferation | BIRC5 | Chr:17q25 |
| 208079_s_at | Proliferation | STK6 | Chr:20q13.2-q13.3 |
| 204092_s_at | Proliferation | STK6 | Chr:20q13.2-q13.3 |
| 209642_at | Proliferation | BUB1 | Chr:2q14 |
| 204962_s_at | Proliferation | CENPA | Chr:2p24-p21 |
| 218355_at | Proliferation | KIF4A | Chr:Xq13.1 |
| 209408_at | Proliferation | KIF2C | Chr:1p34.1 |
| 202870_s_at | Proliferation | CDC20 | Chr:1p34.1 |
| 202580_x_at | Proliferation | FOXM1 | Chr:12p13 |
| 209714_s_at | Proliferation | CDKN3 | Chr:14q22 |
| 203764_at | Proliferation | DLG7 | Chr:14q22.1 |
| 203554_x_at | Proliferation | PTTG1 | Chr:5q35.1 |
| 214710_s_at | Proliferation | CCNB1 | Chr:5q12 |
| 210559_s_at | Proliferation | CDC2 | Chr:10q21.1 |
| 203214_x_at | Proliferation | CDC2 | Chr:10q21.1 |
| 203213_at | Proliferation | CDC2 | Chr:10q21.1 |
| 206102_at | Proliferation | KIAA0186 | Chr:20p11.1 |
| 218726_at | Proliferation | DKFZp762E1312 | Chr:2q37.1 |
| 213226_at | Proliferation | PMSCL1 | Chr:4q27 |
| 203362_s_at | Proliferation | MAD2L1 | Chr:4q27 |
| 203418_at | Proliferation | CCNA2 | Chr:4q25-q31 |
| 219918_s_at | Proliferation | ASPM | Chr:1q31 |
| 204641_at | Proliferation | NEK2 | Chr:1q32.2-q41 |
| 207828_s_at | Proliferation | CENPF | Chr:1q32-q41 |
| 206364_at | Proliferation | KIF14 | Chr:1pter-q31.3 |
| 204822_at | Proliferation | TTK | Chr:6q13-q21 |
| 204162 at | Proliferation | HEC | Chr:18p11.31 |
| 204033_at | Proliferation | TRIP13 | Chr:5p15.33 |
| 212022_s_at | Proliferation | MKI67 | Chr:10q25-qter |
| 205046_at | Proliferation | CENPE | Chr:4q24-q25 |
| 219148_at | Proliferation | TOPK | Chr:8p21.2 |
| 219978_s_at | Proliferation | ANKT | Chr:15q14 |
| 218883_s_at | Proliferation | FLJ23468 | Chr:4q35.1 |
| 209773_s_at | Proliferation | RRM2 | Chr:2p25-p24 |
| 201890_at | Proliferation | RRM2 | Chr:2p25-p24 |
| 204026_s_at | Proliferation | ZWINT | Chr:10q21-q22 |
| 202503_s_at | Proliferation | KIAA0101 | Chr:15q22.1 |
| 203145_at | Proliferation | SPAG5 | Chr:17q11.1 |
| 201292_at | Proliferation | TOP2A | Chr:17q21-q22 |
| 201291_s_at | Proliferation | TOP2A | Chr:17q21-q22 |
| 207165 at | Proliferation | HMMR | Chr:5q33.2-qter |
| 218663_at | Proliferation | HCAP-G | Chr:4p16-p15 |
| 209464 at | Proliferation | STK12 | Chr:17p13.1 |
| 221436_s_at | Proliferation | GRCC8 | Chr:12p13 |
| 202779_s_at | Proliferation | E2-EPF | Chr:19q13.43 |
| 220651_s_at | Proliferation | MCM10 | Chr:10p13 |
| 205394_at | Proliferation | CHEK1 | Chr:11q24-q24 |
| 205393_s_at | Proliferation | CHEK1 | Chr:11q24-q24 |
| 212949 at | Proliferation | BRRN1 | Chr:2q11.2 |
| 204146_at | Proliferation | PIR51 | Chr:12p13.2-p13.1 |
| 204023_at | Proliferation | RFC4 | Chr:3q27 |
| 202107_s_at | Proliferation | MCM2 | Chr:3q21 |
| 202589_at | Proliferation | TYMS | Chr:18p11.32 |
| 219555_s_at | Proliferation | BM039 | Chr:16q23.1 |
| 202094 at | Proliferation | BIRC5 | Chr:17q25 |
| 204603 at | Proliferation | EXO1 | Chr:1q42-q43 |
| 204170_s_at | Proliferation | CKS2 | Chr:9q22 |
| 203358_s_at | Proliferation | EZH2 | Chr:7q35-q36 |
| 203276 at | Proliferation | LMNB1 | Chr:5q23.3-q31.1 |
| 201710_at | Proliferation | MYBL2 | Chr:20q13.1 |
| 218585_s_at | Proliferation | RAMP | --- |
| 218308_at | Proliferation | TACC3 | Chr:4p16.3 |
| 211814_s_at | Proliferation | CCNE2 | Chr:8q22.1 |
| 205034_at | Proliferation | CCNE2 | Chr:8q22.1 |
| 219000_s_at | Proliferation | MGC5528 | Chr:8q24.12 |
| 203046_s_at | Proliferation | TIMELESS | Chr:12q12-q13 |
| 202338_at | Proliferation | TK1 | Chr:17q23.2-q25.3 |
| 220295_x_at | Proliferation | FLJ20354 | Chr:1p31.2 |
| 206632_s_at | Proliferation | APOBEC3B | Chr:22q13.1-q13.2 |
| 204318_s_at | Proliferation | GTSE1 | Chr:22q13.2-q13.3 |
| 213008_at | Proliferation | FLJ10719 | Chr:15q25-q26 |
| 202240_at | Proliferation | PLK | Chr:16p12.3 |
| 219493_at | Proliferation | SHCBP1 | Chr:16q11.2 |
| 219105_x_at | Proliferation | ORC6L | Chr:16q 12 |
| 221521_s_at | Proliferation | LOC51659 | Chr:16q24.1 |
| 203968_s_at | Proliferation | CDC6 | Chr:17q21.3 |
| 203967_at | Proliferation | CDC6 | Chr:17q21.3 |
| 209916_at | Proliferation | KIAA1630 | Chr:10p14 |
| 205436_s_at | Proliferation | H2AFX | Chr:11q23.2-q23.3 |
| 221922_at | Proliferation | LGN | Chr:1p13.2 |
| 205240_at | Proliferation | LGN | Chr:1p13.2 |
| 218741 at | Proliferation | MGC861 | Chr:22q13.2 |
| 216237_s_at | Proliferation | MCM5 | Chr:22q13.1 |
| 201755_at | Proliferation | MCM5 | Chr:22q13.1 |
| 209832_s_at | Proliferation | CDT1 | Chr:16q24.3 |

In a preferred embodiment of the invention, said first marker gene is TOP2A. In another specific embodiment of the invention said first marker gene is a gene co-regulated with TOP2A. Co-regulation of two genes, according to the invention, is preferably exemplified by a correlation coefficient between expression levels of said two genes in multiple tissue samples of greater than 0.5, 0.7, 0.9, 0.95, 0.99, or, most preferably 1. The statistical accuracy of the determination of said correlation coefficient is preferably +/- 0.1 (absolute standard deviation).

In a preferred embodiment of the invention, a proliferation metagene expression value is constructed using 2, 3, 4, 5, 10, 20, 50, or all of the genes listed in Table 1.

In a preferred embodiment of the invention, a proliferation metagene expression value is constructed using 2, 3, 4, 5 or 6 genes from the list of TOP2A, UBE2C, STK6, CCNE2, MKI67, or CCNB1.

"Proliferation metagene expression value", within the meaning of the invention, shall be understood to be a calculated gene expression value representing the proliferative activity of a tumor. In a preferred embodiment of the invention, the proliferation metagene expression value is calculated from multiple marker genes selected from Table 1.

A metagene expression value, in this context, is to be understood as being the median of the normalized expression of multiple marker genes. Normalization of the expression of multiple marker genes is preferably achieved by dividing the expression level of the individual marker genes to be normalized by the respective individual median expression of these marker genes (per gene normalization), wherein said median expression is preferably calculated from multiple measurements of the respective gene in a sufficiently large cohort of test individuals. The test cohort preferably comprises at least 3, 10, 100, or 200 individuals.

Preferably, the calculation of the proliferation metagene expression value is performed by:
i) determining the gene expression value of at least two, preferably more genes from the list of table 1
ii) "normalizing" the gene expression value of each individual gene by dividing the expression value with a coefficient which is approximately the median expression value of the respective gene in a representative node negative breast cancer cohort
iii) calculating the median of the group of normalized gene expression values

The present invention further relates to a prognostic method as defined above, wherein said second marker gene is an immune cell gene or an immune globulin gene. An "immune cell gene" shall be understood to be a gene which is specifically expressed in immune cells, most preferably in T-cells, B-cells or natural killer cells. A gene shall be understood to be specifically expressed in a certain cell type, within the meaning of the invention, if the expression level of said gene in said cell type is at least 2-fold, 5-fold, 10-fold, 100-fold, 1000-fold, or 10000-fold higher than in a reference cell type, or in a mixture of reference cell types. Preferred reference cell types are muscle cells, smooth muscle cells, or non-cancerous breast tissue cells.

Alternatively, an immune cell gene shall be understood as being a gene selected from Table 2. In preferred methods of the invention said second marker gene is selected from Table 2.

Because of the great variability in the primary sequence of immune genes it is conceived that the concept of using metagenes is particularly useful when determining the immune gene status in methods of the invention. Thus, in a preferred embodiment of the invention, the claimed methods use the information on the expression of a single proliferation marker gene (preferably selected from Table 1), but information on the expression of multiple immune genes (preferably selected from Table 2), e.g., an immune system metagene expression is applied.

In further preferred embodiments of the invention, the expression level of multiple first and second marker genes are determined in steps (b) and (d), and a comparison step between the multiple first and the multiple second marker genes is performed by a "majority voting algorithm".

In a majority voting algorithm, according to the invention, a suitable threshold level is first determined for each individual first and second marker gene used in the method. The suitable threshold level can be determined from measurements of the marker gene expression in multiple individuals from a test cohort. Preferably, the median expression of the first said marker gene in said multiple expression measurements is taken as the suitable threshold value for the first said marker gene. Preferably, the third quartile expression of the second said marker gene in said multiple expression measurements is taken as the suitable threshold value for the second said marker gene.

In a majority voting algorithm, the comparison of multiple marker genes with a threshold level is performed as follows:
1. The individual marker genes are compared to their respective threshold levels.
2. The number of marker genes, the expression level of which is above their respective threshold level, is determined.
3. If a sufficiently large number of marker genes is expressed above their respective threshold level, then the expression level of the multiple marker genes is taken to be "above the threshold level".

"A sufficiently large number", in this context, means preferably 30%, 50%, 80%, 90%, or 95% of the marker genes used.

Because of the great variability in the primary sequence of immune genes it is conceived that the concept "majority voting" is particularly useful when determining the immune gene status in methods of the invention. Thus, in a preferred embodiment of the invention, the claimed methods use the information on the expression of a single proliferation marker gene (preferably selected from Table 1), but information on the expression of multiple immune genes (preferably selected from Table 2) is compared to a threshold level using a majority voting algorithm.

**Table 2:**

| **Probe Set** | **Classification** | **Gene Symbol** | **Location** |
|---|---|---|---|
| 1405_i_at | Cellular Immunsystem | CCL5 | Chr:17q11.2-q12 |
| 201422_at | Cellular Immunsystem | IFI30 | Chr:19p13.1 |
| 201487_at | Cellular Immunsystem | CTSC | Chr:11q14.1-q14.3 |
| 201858_s_at | Cellular Immunsystem | PRG1 | Chr:10q22.1 |
| 202269_x_at | Cellular Immunsystem | GBP1 | Chr:1p22.2 |
| 202270_at | Cellular Immunsystem | GBP1 | Chr:1p22.2 |
| 202307_s_at | Cellular Immunsystem | TAP1 | Chr:6p21.3 |
| 202524_s_at | Cellular Immunsystem | SPOCK2 | Chr:10pter-q25.3 |
| 202644_s_at | Cellular Immunsystem | TNFAIP3 | Chr:6q23 |
| 202901_x_at | Cellular Immunsystem | CTSS | Chr:1q21 |
| 202902_s_at | Cellular Immunsystem | CTSS | Chr:1q21 |
| 202953_at | Cellular Immunsystem | C1QB | Chr:1p36.3-p34.1 |
| 203185_at | Cellular Immunsystem | RASSF2 | Chr:20pter-p12.1 |
| 203470_s_at | Cellular Immunsystem | PLEK | Chr:2p13.2 |
| 203471_s_at | Cellular Immunsystem | PLEK | Chr:2p13.2 |
| 203645_s_at | Cellular Immunsystem | CD163 | Chr:12p13.3 |
| 203760_s_at | Cellular Immunsystem | SLA | Chr:8q24 |
| 203828_s_at | Cellular Immunsystem | NK4 | Chr:16p13.3 |
| 203868_s_at | Cellular Immunsystem | VCAM1 | Chr:1p32-p31 |
| 203915_at | Cellular Immunsystem | CXCL9 | Chr:4q21 |
| 204116_at | Cellular Immunsystem | IL2RG | Chr:Xq13.1 |
| 204118 at | Cellular Immunsystem | CD48 | Chr:1q21.3-q22 |
| 204192_at | Cellular Immunsystem | CD37 | Chr:19p13-q13.4 |
| 204198_s_at | Cellular Immunsystem | RUNX3 | Chr:1p36 |
| 204205_at | Cellular Immunsystem | APOBEC3G | Chr:22q13.1-q13.2 |
| 204279_at | Cellular Immunsystem | PSMB9 | Chr:6p21.3 |
| 204533_at | Cellular Immunsystem | CXCL10 | Chr:4q21 |
| 204563_at | Cellular Immunsystem | SELL | Chr:1q23-q25 |
| 204655_at | Cellular Immunsystem | CCL5 | Chr:17q11.2-q12 |
| 204661_at | Cellular Immunsystem | CDW52 | Chr:1p36 |
| 204834_at | Cellular Immunsystem | FGL2 | Chr:7q11.23 |
| 204882_at | Cellular Immunsystem | KIAA0053 | Chr:2p13.2 |
| 204890_s_at | Cellular Immunsystem | LCK | Chr:1p34.3 |
| 204891_s_at | Cellular Immunsystem | LCK | Chr:1p34.3 |
| 204923_at | Cellular Immunsystem | CXorf9 | Chr:Xq26 |
| 204959_at | Cellular Immunsystem | MNDA | Chr:1q22 |
| 205038_at | Cellular Immunsystem | ZNFN1A1 | Chr:7p13-p11.1 |
| 205098_at | Cellular Immunsystem | CCR1 | Chr:3p21 |
| 205159_at | Cellular Immunsystem | CSF2RB | Chr:22q13.1 |
| 205269_at | Cellular Immunsystem | LCP2 | Chr:5q33.1-qter |
| 205419_at | Cellular Immunsystem | EBI2 | Chr:13q32.2 |
| 205488_at | Cellular Immunsystem | GZMA | Chr:5q11-q12 |
| 205495_s_at | Cellular Immunsystem | GNLY | Chr:2p12-q11 |
| 205569_at | Cellular Immunsystem | LAMP3 | Chr:3q26.3-q27 |
| 205671_s_at | Cellular Immunsystem | HLA-DOB | Chr:6p21.3 |
| 205681_at | Cellular Immunsystem | BCL2A1 | Chr:15q24.3 |
| 205758_at | Cellular Immunsystem | CD8A | Chr:2p12 |
| 205798_at | Cellular Immunsystem | IL7R | Chr:5p13 |
| 205821_at | Cellular Immunsystem | D12S2489E | Chr:12p13.2-p12.3 |
| 205831_at | Cellular Immunsystem | CD2 | Chr:1p13 |
| 205861_at | Cellular Immunsystem | SPIB | Chr:19q13.3-q13.4 |
| 205890_s_at | Cellular Immunsystem | UBD | Chr:6p21.3 |
| 205992_s_at | Cellular Immunsystem | IL15 | Chr:4q31 |
| 206134 at | Cellular Immunsystem | ADAMDEC1 | Chr:8p21.1 |
| 206150_at | Cellular Immunsystem | TNFRSF7 | Chr:12p13 |
| 206214_at | Cellular Immunsystem | PLA2G7 | Chr:6p21.2-p12 |
| 206337_at | Cellular Immunsystem | CCR7 | Chr:17q12-q21.2 |
| 206513_at | Cellular Immunsystem | AIM2 | Chr:1q22 |
| 206666_at | Cellular Immunsystem | GZMK | Chr:5q11-q12 |
| 206715_at | Cellular Immunsystem | TFEC | Chr:7q31.2 |
| 206978_at | Cellular Immunsystem | CCR2 | Chr:3p21 |
| 206991_s_at | Cellular Immunsystem | CCR5 | Chr:3p21 |
| 207238_s_at | Cellular Immunsystem | PTPRC | Chr:1q31-q32 |
| 207339_s_at | Cellular Immunsystem | LTB | Chr:6p21.3 |
| 207419_s_at | Cellular Immunsystem | RAC2 | Chr:22q13.1 |
| 207677_s_at | Cellular Immunsystem | NCF4 | Chr:22q13.1 |
| 207697_x_at | Cellular Immunsystem | LILRB2 | Chr:19q13.4 |
| 208018_s_at | Cellular Immunsystem | HCK | Chr:20q11-q12 |
| 208885_at | Cellular Immunsystem | LCP1 | Chr:13q14.3 |
| 209083_at | Cellular Immunsystem | CORO1A | Chr:16p11.2 |
| 209606_at | Cellular Immunsystem | PSCDBP | Chr:2q11.2 |
| 209670_at | Cellular Immunsystem | TRA@ | Chr:14q11.2 |
| 209671_x_at | Cellular Immunsystem | TRA@ | Chr:14q11.2 |
| 209685_s_at | Cellular Immunsystem | PRKCB1 | Chr:16p11.2 |
| 209795_at | Cellular Immunsystem | CD69 | Chr:12p13-p12 |
| 209823_x_at | Cellular Immunsystem | HLA-DQB1 | Chr:6p21.3 |
| 209901_x_at | Cellular Immunsystem | AIF1 | Chr:6p21.3 |
| 209949_at | Cellular Immunsystem | NCF2 | Chr:1q25 |
| 209969_s_at | Cellular Immunsystem | STAT1 | Chr:2q32.2 |
| 210031_at | Cellular Immunsystem | CD3Z | Chr:1q22-q23 |
| 210140 at | Cellular Immunsystem | CST7 | Chr:20p11.21 |
| 210163_at | Cellular Immunsystem | CXCL11 | Chr:4q21.2 |
| 210164 at | Cellular Immunsystem | GZMB | Chr:14q11.2 |
| 210538 s at | Cellular Immunsystem | BIRC3 | Chr:11q22 |
| 210895_s_at | Cellular Immunsystem | CD86 | Chr:3q21 |
| 210915_x_at | Cellular Immunsystem | TRB@ | Chr:7q34 |
| 210972_x_at | Cellular Immunsystem | TRA@ | Chr:14q11.2 |
| 211122_s_at | Cellular Immunsystem | CXCL11 | Chr:4q21.2 |
| 211336_x_at | Cellular Immunsystem | LILRB1 | Chr:19q13.4 |
| 211339_s_at | Cellular Immunsystem | ITK | Chr:5q31-q32 |
| 211367_s_at | Cellular Immunsystem | CASP1 | Chr:11q23 |
| 211368_s_at | Cellular Immunsystem | CASP1 | Chr:11q23 |
| 211656_x_at | Cellular Immunsystem | HLA-DQB1 | Chr:6p21.3 |
| 211742_s_at | Cellular Immunsystem | EVI2B | Chr:17q11.2 |
| 211795_s_at | Cellular Immunsystem | FYB | Chr:5p13.1 |
| 211796_s_at | Cellular Immunsystem | TRB@ | Chr:7q34 |
| 211902_x_at | Cellular Immunsystem | TRA@ | Chr:14q11.2 |
| 212587_s_at | Cellular Immunsystem | PTPRC | Chr:1q31-q32 |
| 212588_at | Cellular Immunsystem | PTPRC | Chr:1q31-q32 |
| 212671_s_at | Cellular Immunsystem | HLA-DQA1 | Chr:6p21.3 |
| 213095_x_at | Cellular Immunsystem | AIF1 | Chr:6p21.3 |
| 213193 x at | Cellular Immunsystem | TRB@ | Chr:7q34 |
| 213539_at | Cellular Immunsystem | CD3D | Chr:11q23 |
| 213603_s_at | Cellular Immunsystem | RAC2 | Chr:22q13.1 |
| 213888_s_at | Cellular Immunsystem | --- | --- |
| 213915_at | Cellular Immunsystem | NKG7 | Chr:19q13.33 |
| 213958_at | Cellular Immunsystem | CD6 | Chr:11q13 |
| 213975_s_at | Cellular Immunsystem | LYZ | Chr:12q14.3 |
| 214038_at | Cellular Immunsystem | CCL8 | Chr:17q11.2 |
| 214054_at | Cellular Immunsystem | DOK2 | Chr:8p21.2 |
| 214084_x_at | Cellular Immunsystem | NCF1 | Chr:7q11.23 |
| 214560_at | Cellular Immunsystem | FPRL2 | Chr:19q13.3-q13.4 |
| 214617_at | Cellular Immunsystem | PRF1 | Chr:10q22 |
| 214995_s_at | Cellular Immunsystem | KA6 | Chr:22q13.1 |
| 215049_x_at | Cellular Immunsystem | CD163 | Chr:12p13.3 |
| 215051_x_at | Cellular Immunsystem | AIF1 | Chr:6p21.3 |
| 216598_s_at | Cellular Immunsystem | CCL2 | Chr:17q11.2-q21.1 |
| 217143_s_at | Cellular Immunsystem | TRD@ | Chr:14q11.2 |
| 218232_at | Cellular Immunsystem | C1QA | Chr:1p36.3-p34.1 |
| 219014_at | Cellular Immunsystem | PLAC8 | Chr:4q21.3 |
| 219385_at | Cellular Immunsystem | BLAME | Chr:1q22 |
| 219386_s_at | Cellular Immunsystem | BLAME | Chr:1q22 |
| 219505_at | Cellular Immunsystem | CECR1 | Chr:22q11.2 |
| 219528_s_at | Cellular Immunsystem | BCL11B | Chr:14q32.31 |
| 219607_s_at | Cellular Immunsystem | MS4A4A | Chr:11q12 |
| 219812_at | Cellular Immunsystem | MGC2463 | Chr:7q22.1 |
| 220330_s_at | Cellular Immunsystem | SAMSN1 | Chr:21q11 |
| 220485_s_at | Cellular Immunsystem | SIRPB2 | Chr:20p13 |
| 220577_at | Cellular Immunsystem | FLJ13373 | Chr:11p15.4 |
| 221210_s_at | Cellular Immunsystem | C1orf13 | Chr:1q25 |
| 221698_s_at | Cellular Immunsystem | CLECSF12 | Chr:12p13.2-p12.3 |
| 34210_at | Cellular Immunsystem | CDW52 | Chr:1p36 |
| 37145_at | Cellular Immunsystem | GNLY | Chr:2p12-q11 |
| 44790_s_at | Cellular Immunsystem | C13orf18 | Chr:13q14.11 |
| 205267_at | Humoral Immunsystem | POU2AF1 | Chr:11q23.1 |
| 205692_s_at | Humoral Immunsystem | CD38 | Chr:4p15 |
| 209138_x_at | Humoral Immunsystem | IGLJ3 | Chr:22q11.1-q11.2 |
| 209374_s_at | Humoral Immunsystem | IGHM | Chr:14q32.33 |
| 211430_s_at | Humoral Immunsystem | IGHG3 | Chr:14q32.33 |
| 211633_x_at | Humoral Immunsystem | ICAP-1A | Chr:2p25.2 |
| 211634_x_at | Humoral Immunsystem | IGHG3 | Chr:14q32.33 |
| 211635_x_at | Humoral Immunsystem | IGHG3 | Chr:14q32.33 |
| 211637_x_at | Humoral Immunsystem | IGHM | Chr:14q32.33 |
| 211641_x_at | Humoral Immunsystem | IGHM | Chr:14q32.33 |
| 211643_x_at | Humoral Immunsystem | IGKC | Chr:2p12 |
| 211644_x_at | Humoral Immunsystem | IGKC | Chr:2p12 |
| 211645_x_at | Humoral Immunsystem | IGKC | Chr:2p12 |
| 211650_x_at | Humoral Immunsystem | IGHM | Chr:14q32.33 |
| 211798_x_at | Humoral Immunsystem | IGLJ3 | Chr:22q11.1-q11.2 |
| 211868_x_at | Humoral Immunsystem | --- | --- |
| 211881_x_at | Humoral Immunsystem | IGLJ3 | Chr:22q11.1-q11.2 |
| 211908_x_at | Humoral Immunsystem | IGHM | Chr:14q32.33 |
| 212311_at | Humoral Immunsystem | KIAA0746 | Chr:4p15.2 |
| 212314_at | Humoral Immunsystem | KIAA0746 | Chr:4p15.2 |
| 212592_at | Humoral Immunsystem | IGJ | Chr:4q21 |
| 213502_x_at | Humoral Immunsystem | LOC91316 | Chr:22q11.21 |
| 214669_x_at | Humoral Immunsystem | IGKC | Chr:2p12 |
| 214677_x_at | Humoral Immunsystem | IGLJ3 | Chr:22q11.1-q11.2 |
| 214768_x_at | Humoral Immunsystem | IGKC | Chr:2p12 |
| 214777_at | Humoral Immunsystem | IGKC | Chr:2p12 |
| 214836_x_at | Humoral Immunsystem | IGKC | Chr:2p12 |
| 214916_x_at | Humoral Immunsystem | IGHM | Chr:14q32.33 |
| 214973_x_at | Humoral Immunsystem | IGHG3 | Chr:14q32.33 |
| 215118_s_at | Humoral Immunsystem | --- | --- |
| 215121_x_at | Humoral Immunsystem | IGLJ3 | Chr:22q11.1-q11.2 |
| 215176_x_at | Humoral Immunsystem | IGKC | Chr:2p12 |
| 215214_at | Humoral Immunsystem | IGL@ | Chr:22q11.1-q11.2 |
| 215379_x_at | Humoral Immunsystem | IGLJ3 | Chr:22q11.1-q11.2 |
| 215946_x_at | Humoral Immunsystem | LOC91316 | Chr:22q11.21 |
| 215949_x_at | Humoral Immunsystem | --- | --- |
| 216207_x_at | Humoral Immunsystem | IGKV1D-13 | Chr:2p12 |
| 216365_x_at | Humoral Immunsystem | IGLJ3 | Chr:22q11.1-q11.2 |
| 216401_x_at | Humoral Immunsystem | --- | --- |
| 216412_x_at | Humoral Immunsystem | IGL@ | Chr:22q11.1-q11.2 |
| 216491_x_at | Humoral Immunsystem | IGHM | Chr:14q32.33 |
| 216510_x_at | Humoral Immunsystem | --- | --- |
| 216542_x_at | Humoral Immunsystem | --- | --- |
| 216557 x at | Humoral Immunsystem | --- | --- |
| 216560 x at | Humoral Immunsystem | IGL@ | Chr:22q11.1-q11.2 |
| 216576_x_at | Humoral Immunsystem | --- | --- |
| 216853_x_at | Humoral Immunsystem | IGLJ3 | Chr:22q11.1-q11.2 |
| 216984_x_at | Humoral Immunsystem | IGLJ3 | Chr:22q11.1-q11.2 |
| 217022_s_at | Humoral Immunsystem | MGC27165 | Chr:14 |
| 217148_x_at | Humoral Immunsystem | IGLJ3 | Chr:22q11.1-q11.2 |
| 217157_x_at | Humoral Immunsystem | IGKC | Chr:2p12 |
| 217179_x_at | Humoral Immunsystem | IGL@ | Chr:22q11.1-q11.2 |
| 217227_x_at | Humoral Immunsystem | IGL@ | Chr:22q11.1-q11.2 |
| 217235_x_at | Humoral Immunsystem | IGLJ3 | Chr:22q11.1-q11.2 |
| 217236_x_at | Humoral Immunsystem | IGHM | Chr:14q32.33 |
| 217258 x at | Humoral Immunsystem | --- | --- |
| 217281_x_at | Humoral Immunsystem | IGHG3 | Chr:14q32.33 |
| 217378_x_at | Humoral Immunsystem | --- | --- |
| 217480_x_at | Humoral Immunsystem | --- | --- |
| 221286_s_at | Humoral Immunsystem | PACAP | Chr:5q23-5q31 |

In specific embodiments of the invention, a single, or 2, 5, 10, 20, 50 or 100 second marker genes are used.

In preferred methods of the invention, said second marker gene is IGHG or a gene co-regulated with IGHG.

In preferred methods of the invention, said second marker gene is IGHG3 or a gene co-regulated with IGHG3.

In a preferred embodiment of the invention, an immune system metagene expression value is constructed using 2, 3, 4, 5, 10, 20, 50, or all of the genes listed in Table 2.

In a preferred embodiment of the invention, an immune system metagene expression value is constructed using 2, 3, or 4 genes from the list of IGHG, IGHG3, IGKC, IGLJ3, IGHM.

Preferably, the calculation of an immune system metagene is done by
1. determining the gene expression value of at least two, preferably more genes from the list of table 2
2. "normalizing" the gene expression value of each individual gene by dividing the expression value with a coefficient which is approximately the median expression value of the respective gene in a representative node negative breast cancer cohort
3. calculating the median of the group of normalized gene expression values

In preferred methods of the invention, the determination of expression levels is on a gene chip, e.g. on an Affymetrix™ gene chip.

In another preferred method of the invention, the determination of expression levels is done by kinetic real time PCR.

The present invention further relates to a system for performing methods of the current invention, said system comprising
(a) means for storing data on the nodal status of said patient;
(b) means for determining the expression level of at least one first marker gene;
(c) means for comparing said expression level of said first marker gene with a predetermined first threshold value;
(d) means for determining the expression level of at least one second marker gene; and
(e) computing means programmed to give a favorable prognosis if said data on said nodal status indicates a negative nodal status and said comparison of said expression level of said first marker gene with said predetermined first threshold value indicates a fast proliferating tumor and said expression level of said second marker gene is above a predetermined second threshold level, and
said computing means being programmed to give an unfavorable prognosis if said information on said nodal status indicates a negative nodal status and said comparison of said expression level of said first marker gene with said predetermined first threshold value indicates a fast proliferating tumor and said expression level of said second marker gene is below a predetermined second threshold level.

The person skilled in the art readily appreciates that a favorable prognosis can be given if said expression level of said first marker gene with said predetermined first threshold value indicates a *slow* proliferating tumor. According to the invention, this is independent of the expression level determined for the second marker gene. Methods of the invention as described above can be modified accordingly.

In preferred systems of the invention, said prognosis is an estimation of the likelihood of metastasis free survival over a predetermined period of time.

In preferred methods of the invention, the expression of said first marker gene is indicative of fast proliferation of the tumor.

In preferred systems of the invention, said first marker gene is selected from Table 1.

In preferred systems of the invention, said first marker gene is TOP2A. In other preferred systems of the invention, said first marker gene is a gene co-regulated with TOP2A.

In preferred systems of the invention, said second marker gene is an immune cell gene, or is an immune globulin gene. Preferred second marker genes are expressed specifically in T-cells or in B-cells or in natural killer cells.

In preferred systems of the invention, said second marker gene is selected from Table 2. In particularly preferred systems of the invention, said second marker gene is IGHG3 or a gene co-regulated with IGHG3.

In preferred systems of the invention, the determination of expression levels is on a gene chip.

### Example

We analyzed 200 node-negative breast cancers not treated with systemic therapy using PCA, a method also described by Alter and co-workers (2000) as singular value decomposition. This method allows for extracting information from high-dimensional datasets. It is well accepted, that the top few principal components identify broad characteristics of the data (Roden et al, 2006). To ensure an optimal visualization of the tumors depending on their most important principal components (PC), we used PC 1-3. Samples are separated on PC1 predominantly according to the expression of the ER metagene. This again underlines the pivotal influence of ER for the molecular profile of breast cancer. The proliferation metagene forms another axis. All ER negative breast cancer samples are characterized by high proliferation. However, samples scored as ER positive by immunohistochemistry showed differences in both, extend of expression of ER co-regulated genes as well as in the extend of proliferation. Interestingly, tumors with intermediate ER expression showed the biggest variation in proliferative activity. High expression of proliferation associated genes in this subtype was linked with similar bad prognosis as for ER negative tumors, indicating that proliferation is the strongest outcome predictor in untreated node negative breast cancer patients. When systematically utilizing different metagenes for an explanation for the noticeable paucity of early metastases in the region with concurrent low ER and high proliferation, we detected a third axis. This axis is almost perpendicular to the proliferation axis. It is formed of the B cell metagene, containing B cell associated genes like immunoglobulines and to a lesser extent the T cell metagene, containing T cell related genes like the T cell receptor (TCR). These two metagenes are largely overlapping. In the region of high expression of these metagenes, only rare metastases occur despite high proliferation and low ER expression.

Gene expression patterns of 200 node-negative breast cancer patients which were not treated in the adjuvant setting, were recorded with the Affymetrix HG-U133A array. After performing an unsupervised hierarchical cluster analysis using 2579 genes selected for variable expression within our dataset, metagenes were constructed for the different cluster. These metagenes were then visualized in a principle component analysis (PCA). The prognostic impact was assessed with univariate statistics. The prognostic power of the method was confirmed with a previously published dataset (Wang et al, 2005).

Using unsupervised hierarchical cluster analysis, several different gene clusters were detected. These could roughly be categorized as basal-like, T-cell, B-cell, interferon, proliferation, estrogen regulated, chromosome 17 (ERBB2), stromal, normal-like (adipocyte), Jun-Fos, and transcription cluster. Visualizing ER and proliferation clusters as well as time to metastasis (TTM) with PCA showed discrete patterns which were highly reproducible in the validation cohort. Both B cell and T cell metagene yielded additional information and had significant prognostic value, in particular, in rapidly proliferating tumors. For the B cell metagene the prognostic value could be independently confirmed in the validation cohort.

We could confirm in two independent cohorts of untreated node-negative breast cancer patients, that especially the humoral immune system plays a pivotal role for the metastasis-free survival of rapidly proliferating tumors.

### Patient characteristics and tissue specimens

The population based study cohort consisted of 200 lymph-node negative breast cancer patients treated at the Department of Obstetrics and Gynecology of the Johannes Gutenberg University Mainz between 1988 and 1998. Patients were all treated with surgery and did not receive any systemic therapy in the adjuvant setting. The established prognostic factors (tumor size, age at diagnosis, steroid receptor status) were collected from the original pathology reports of the gynecological pathology division within our department. Grade was defined according to the system of Elston and Ellis.

Patients were treated either with modified radical mastectomy (n=75) or breast conserving surgery followed by irradiation (n=125) and had to be without any evidence of lymph node and distant metastasis at the time of surgery. The median age of the patients at surgery was 60 years (range, 34-89 years). The median time of follow up was 92 months. Within this follow-up period, 68 (34 %) patients relapsed, of these 46 (23 %) developed distant metastases. 28 (14 %) patients died of breast cancer and 26 (13 %) patients died of unrelated reasons.

Frozen sections were taken for histology and the presence of breast cancer was confirmed in all samples. Tumor cell content exceeded 40 % in all cases. Approximately 50 mg of snap frozen breast tumor tissue was crushed in liquid nitrogen. RLT-Buffer was added and the homogenate was spun through a QIAshredder column (QIAGEN, Hilden, Germany). From the eluate total RNA was isolated by the RNeasy Kit (QIAGEN) according to the manufacturer instruction. RNA yield was determined by UV absorbance and RNA quality was assessed by analysis of ribosomal RNA band integrity on an Agilent 2100 Bioanalyzer RNA 6000 LabChip kit (Agilent Technologies, Palo Alto, CA). The study was approved by the ethical review board of the medical association of Rhineland-Palatinate.

**Table 3: Patient characteristics of the Mainz dataset (n = 200) and the published Rotterdam dataset (n = 286). Our collection is population based whereas the Rotterdam cohort was selected for a case control study (Wang et al. 2005).**

| | **Mainz Cohort (n = 200)** | | **Rotterdam Cohort (n = 286)** | |
|---|---|---|---|---|
| **Tumor Size** | | | | |
| T1 | 111 | 56% | 146 | *51%* |
| T2 | 81 | *40%* | 132 | *46%* |
| T3/4 | 8 | 4% | 8 | *3%* |
| **Tumor Grade** | | | | |
| Well differentiated | 41 | 21% | 7 | 2% |
| Moderately differentiated | 110 | *55%* | 42 | *15%* |
| Poor / undifferentiated | 45 | *23%* | 148 | 52% |
| Unknown | 4 | 2% | 89 | *31%* |
| **ERICA (IRS)** | | | DCC or EIA | |
| 0-1 | 44 | 22% | 77 | 27% |
| 2-12 | 156 | *78%* | 209 | *73%* |
| **PRICA (IRS)** | | | DCC or EIA | |
| 0-1 | 70 | *35%* | 111 | *39%* |
| 2-12 | 130 | 65% | 165 | *58%* |
| Unknown | | | 10 | *3%* |
| **Age, years** | | | | |
| Mean (DS) | 60 | (12) | 54 | *(12)* |
| ≤ 40 | 10 | 5% | 36 | *13%* |
| 41 -55 | 64 | *32%* | 129 | 45% |
| 56-70 | 83 | 42% | 89 | *31%* |
| ≥ 70 | 43 | 22% | 32 | *11%* |
| **Metastasis within 5 years** | | | | |
| Yes | 27 | 14% | 93 | *33%* |
| No | 149 | *75%* | 183 | 64% |
| Censored | 24 | 12% | 10 | *3%* |
| Metastasis after 5 years | 19 | 10% | | |

### Determination of the nodal status

Axillary nodal status is the most important prognostic factor in patients with breast cancer. Formal axillary clearance is the best staging procedure, however, it is associated with significant morbidity. About 60% of axillary dissections show no evidence of metastatic disease. As a result, axillary sampling (removal of 4 nodes) has been proposed as an alternative means of assessing nodal status. Staging errors can occur following axillary sampling and this procedure is associated with a higher local recurrence rate. Intra-operative lymph node mapping has been suggested so as to allow identification of the first draining node (the 'sentinel' node) and to reduce the morbidity associated with axillary surgery. In this case the node is identified by injection of 2.5% Patent Blue dye adjacent to the primary tumour and the axilla is explored approximately10 minutes post-injection. The sentinel node is excised and submitted for both frozen section and paraffin histological assessment. It has been shown that histological examination of this node predicted nodal status in 95% of cases. The presence of tumor cells in the histological specimen can alternatively be determined by detection of tumor cell specific nucleic acids using RT-PCR or related methods. In particular, detection of cytokeratin 19 RNA has been proposed for this purpose (Backus et al. 2005).

### Gene expression profiling

The Affymetrix (Santa Clara, CA, USA) HG-U133A array and GeneChip System™ was used to quantify the relative transcript abundance in the breast cancer tissues. Starting from 5 µg total RNA labelled cRNA was prepared using the Roche Microarray cDNA Synthesis, Microarray RNA Target Synthesis (T7) and Microarray Target Purification Kit according to the manufacturer's instruction. In brief, synthesis of first strand cDNA was done by a T7-linked oligo-dT primer, followed by second strand synthesis. Double-stranded cDNA product was purified and then used as template for an in vitro transcription reaction (IVT) in the presence of biotinylated UTP. Labelled cRNA was hybridized to HG-U133A arrays at 45°C for 16 h in a hybridization oven at a constant rotation (60 r.p.m.) and then washed and stained with a streptavidin-phycoerythrin conjugate using the GeneChip fluidic station. We scanned the arrays at 560 nm using the GeneArray Scanner G2500A from Hewlett Packard. The readings from the quantitative scanning were analysed using the Microarray Analysis Suit 5.0 from Affymetrix. In the analysis settings the global scaling procedure was chosen which multiplied the output signal intensities of each array to a mean target intensity of 500. Samples with suboptimal average signal intensities (i.e., scaling factors > 25) or GAPDH 3'/5'ratios > 5 were relabeled and rehybridized on new arrays. Routinely we obtained over 40 percent present calls per chip as calculated by MAS 5.0.

### Previously published microarray datasets

A breast cancer Affymetrix HG-U133A microarray dataset including patient outcome information was downloaded from the NCBI GEO data repository (http://www.ncbi.nlm.nih.gov/geo/). The data set (GSE2034) represents 180 lymph-node negative relapse free patients and 106 lymph-node negative patients that developed a distant metastasis. None of the patients did receive systemic neoadjuvant or adjuvant therapy.

### Analysis of microarray data

For our unpublished dataset selection of "informative" genes was done using the quality control criteria "absent" or "present" as provided by the Affymetrix software, the absolute median signal intensity and the coefficient of variation of a gene within our dataset. Genes passing the quality control filter of having a "present" call in at least 10 samples, median signal intensity above 75 and a coefficient of variation above 60 % within our dataset were considered to be informative and used for subsequent analysis. For unsupervised analysis we performed average linkage hierarchical clustering on all informative genes and samples using Pearson correlation as implemented in GeneSpring 7.0 software (Agilent Technologies, USA). Principle component analysis was performed using GeneSpring 7.0. Clinical information was visualized as categorical or continues variable and relative gene expression was visualized on a relative scale from red, indicating high expression, to blue, indicating low expression. Gene groups were defined after manual selection of nodes of the gene dendrogram as suggested by the occurrence of cluster regions within the heatmap. A metagene was calculated as representative of all genes contained within one gene cluster based on the normalized expression values within the respective dataset. The genes contained within the proliferation cluster are listed in Table 1 and the genes contained within the immune gene clusters are listed in Table 2.

### ROC curve and survival analysis

ROC curve was calculated for metagene 5a with 176 samples fulfilling the criteria that patients remained at least five years disease free (n = 149) or developed a distant metastasis within five years (n = 27) using GraphPad Prism software (USA). Furthermore, ROC analysis was performed in a sub-cohort of Mainz samples defined by metagene 5a expression > 0.99 using metagene 2 and 3 values, respectively. All identified cut off values were used for the analysis of Rotterdam samples without further adjustment. Life tables were calculated according to the Kaplan-Meier method using GraphPad Prism software. Metastasis-free survival (MFS) was computed from the date of diagnosis to the date of diagnosis of distant metastasis. Survival curves were compared with the Log-rank test. Univariate Cox survival analyses were performed using the Cox proportional hazards model. All tests were performed at a significance level of alpha = 0.05. All p values are two sided.

### Hierarchical cluster analysis and biological motives in breast cancer tissues

Primary tumor tissues from 196 patients with invasive breast carcinoma as well as from four patients with DCIS were analyzed by gene expression profiling using HG U133A oligonucleotide arrays. All patients were node negative and did not receive systemic chemo- or endocrine-therapy after surgery. Details about the population based cohort are given in Table 3.

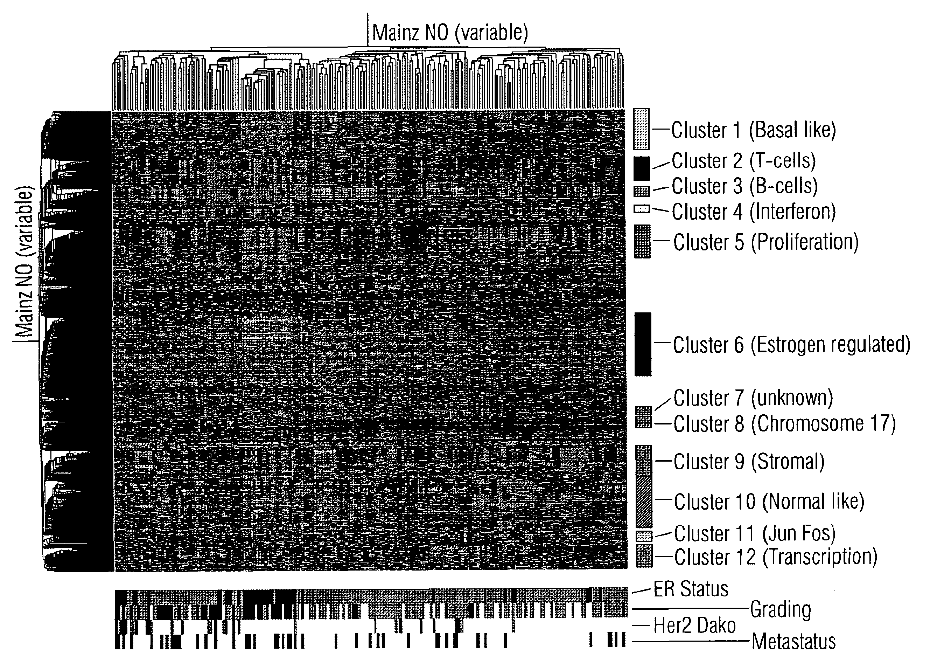

In order to identify co-regulated genes representing distinct biological processes or cell types we performed an unsupervised two dimensional hierarchical cluster analysis using 2579 genes selected for variable expression within our dataset. As seen in the resulting heat map samples as well as genes are grouped according to overall similarity in relative gene expression (figure 1). Several dominant clusters of co-regulated genes become visible and inspection of gene names contained in the individual cluster indicate either the underlying biological process represented by these genes or their cell type specific origin. The clusters can be assigned as basal like, T-cell, B-cell, interferon, proliferation, estrogen regulated, chromosome 17 (ERBB2), stromal, normal like (adipocyte), Jun-Fos, and transcription cluster. Similar clusters have been described by several other groups (Perou et al. 2000, van't Veer et al. 2002). Since estrogen receptor co-regulated genes have a dominant impact on overall gene expression the samples are readily grouped according to their estrogen receptor status as displayed in the sample parameter bar below the heat map (Figure 1). A correlation between tumor grade and expression of proliferation genes might be deduced from the heat map and the sample parameter bar as well. However, any other interrelation between gene expression and clinical or histopathological features of the corresponding tumors are difficult to grasp using hierarchical clustering as visualization method. In particular, the presence of T- or B-cell specific genes is not obviously related with an improved outcome.

### Unsupervised principle component analysis and metagene expression in breast tumors

In order to obtain a clearer view on the molecular heterogeneity of node negative breast cancer we used (unsupervised) principal component analysis (PCA). Since the position of a sample within a PCA plot is determined by its gene expression values, it is of interest to investigate how the relative expression of genes, known to be of relevance for disease outcome contributes to the separation. Proliferation index, tumor grade and estrogen receptor expression have long been recognized to be correlated with disease outcome. Correspondingly, several gene expression profiling studies identified genes involved in certain steps of the cell cycle and estrogen receptor co-regulated genes to be associated with disease outcome. Since we were interested to investigate the complex interrelationships between these biological processes and a potential prognostic role of the immune system we constructed metagenes for the T-cell (metagene 2), B-cell (metagene 3), proliferation (metagene 5a) and estrogen receptor cluster (metagene 6a) by calculating the median of the normalized expression of all genes contained in each respective cluster for each sample.

In our population based cohort samples are separated on principal component 1 (PC1) predominantly according to expression of estrogen receptor 1 (ESR1) and ESR1 co-regulated genes. Accordingly, samples with highest metagene 6a expression cluster on the lower left, those with the lowest values on the lower right. Variable expression is seen in the intermediate area which broadly scatters on PC2. In particular those samples with the lowest metagene 6a values are well separated from all other tumors and appear to constitute a distinct group which may be considered the basal subtype since all samples are PGR and ERBB2 negative and most of them positive for the previously suggested basal like marker KRT5 and KRT17 (data not shown). However, based on the observation that KRT5, KRT17 and other genes proposed as basal like marker genes are expressed in tumors located in a different cluster in the upper region of the PCA these genes are not suited to unequivocally characterize this molecular subtype (data not shown). PC1 in can broadly be considered to form the estrogen receptor axis. Visualization of metagene 5a expression, as indicator of proliferation, in reveals a gradient with samples in the upper left having lowest and samples in the lower right having highest expression. A similar gradient is formed by individual well known cell cycle associated genes like MKI67, CCNE2 and others (data not shown). Therefore, the gradient can be considered to form the proliferation axis. As expected, a high correlation exists between proliferation and tumor grade (data not shown). In addition, expression profiling confirms that tumors of lobular and tubular histology are predominantly estrogen receptor positive and slowly proliferating, whereas ductal tumors highly heterogeneous regarding both. Interestingly, cancers of medullar histology cluster in a region of high proliferation and very low ESR1 expression (data not shown).

When time to distant metastasis is visualized it becomes apparent that most patients suffering an early metastasis are located in the middle and right part along the PC 1- and lower part of the PC2-axis of the plot. These samples are characterized by intermediate to low metagene 6a expression and concurrent high proliferation, i.e. metagene 5a expression. Evidently, two different tumor types are less prone to metastasize, one characterized by very high metagene 6a expression and the other by intermediate metagene 6a and simultaneous low expression of metagene 5a. In a region of samples with relative high proliferation, and low metagene 6a levels a paucity of samples with distant metastasis is observed as well. Interestingly, this region is characterized by high expression of metagene 2 (T-cells) and metagene 3 (B-cells), indicating that a lymphoid infiltration in these tumor tissues might be associated with good outcome. Metagene 2 contains information from gene like T-cell receptor TRA@, TRB@ as well as several other genes preferentially expressed in T-cells, whereas metagene 3 is primarily formed by immunoglobulin heavy and light chain genes of several immunoglobulin classes like IGKC, IGHG3, IGHM. Both metagenes form another gradient within the samples in the PCA plot with an axis from the upper right to the lower left. The complete absence of lymphoid infiltrates in the group of highest metagene 6a expression results in a kind of sandwich situation in which good outcome coincides with either very high or virtually no lymphoid infiltration whereas a particular group with intermediate lymphoid infiltration has a high risk of recurrence.

### Prognostic relevance of lymphoid infiltration in fast proliferating tumors

Since it appears that the immune system does not play a positive role in all breast cancer subtypes we sought to identify the subgroup of patients in which the presence of immune cells is linked with an improved prognosis. From the findings above we reasoned that a protective effect of the immune system might be confined to fast proliferating tumors. Therefore, we performed a ROC analysis for metagene 5a values in order to find a suitable cut off for identification of tumors that develop a distant metastasis within five years i.e. high risk tumors (n = 27) versus those that remained disease free for at least five years (n = 149). The resulting area under the ROC curve was 0.744 (CI 0.631 to 0.856, p < 0.0001) with 81.5 % sensitivity and 56 % specificity at 0.99 as cut off which classified 98 tumors into the high risk category. When we performed a Kaplan Meier survival analysis within this high risk patient sub-cohort which we now stratified according high or low expression of metagene 2 (T-cell) respectively metagene 3 (B-cell) a significant disease free survival benefit was seen for tumors with high metagene 2 expression (hazard ratio 2.77, CI 1.27 to 5.28, p = 0.0088), as well as for high metagene 3 expression (hazard ratio 2.63, CI 1.26 to 3.69, p = 0.0048). In order to test our hypotheses in an independent patient cohort we analyzed a public available expression dataset of node negative untreated breast cancer patients profiled by the same platform as our samples (Wang et al. 2005). A PCA plot was generated using the expression values of all 2579 genes found to be variably expressed in our dataset. Metagenes for estrogen receptor co-regulated genes, proliferation associated genes and the T-cell and B-cell clusters were calculated using the same probesets as used for the Mainz cohort. Kaplan Meier survival analysis was performed using the same cut offs as defined in our finding cohort. The chosen cut off criteria did not yield a separation of high versus low metagene 2 (T-cell cluster) expressing samples (cut off 1.35) in fast proliferating (cut off 0.99) tumors at a significant level (p = 0.2). However, tumors expressing metagene 3, i.e. B-cell related genes, at a cut off above 1.95 had a significant better outcome (p = 0.0048) compared with tumors expressing metagene 2 at low levels.

We could build upon these intriguing findings and were for the first time able to prove a strong association of the expression of the B cell metagene with metastasis-free survival of rapidly proliferating node-negative breast cancer. Based on the findings mentioned above, an antigen-specific humoral immune response could serve as an explanation for the improved survival of rapidly proliferating tumors in our cohort. To validate our findings in a separate cohort, we used a previously published cohort which was also analyzed with the Affymetrix Human U133a gene chip (Wang et al, 2005). Similar to ours, this dataset consists only of untreated node-negative breast cancer patients. These features make the two datasets comparable and allow for estimation of pure prognostic effects without a possible "dilution" by predictive effects. The influence of the B cell metagene was unequivocally confirmed in this separate cohort.

In conclusion, we could confirm in two independent cohorts of untreated node-negative breast cancer patients, that especially the humoral immune system plays a pivotal role for the metastasis-free survival of rapidly proliferating tumors. Further studies are needed to clarify the precise nature of the immunological defense, its failure in certain tumors and to explain its apparent complete lack despite good outcome in others. Extending knowledge about the complex role of immune cells and their interaction in breast cancer tissues should ultimately pave the way for the long awaited successful development of therapeutics aiming at the third prognosis axis.

### REFERENCES

1) Aaltomaa S, Lipponen P, Eskelinen M, Kosma VM, Marin S, Alhava E, Syrjanen K. Lymphocyte infiltrates as a prognostic variable in female breast cancer. Eur J Cancer 28: 859-864, 1992
2) Alter O, Brown PO, Botstein D. Singular value decomposition for genome-wide expression data processing and modeling. Proc Natl Acas Sci USA 97: 10101-10106, 2000
3) Backus, J. et al. Identification and characterization of optimal gene expression markers for detection of breast cancer metastasis. JMD 7, 327-336: 2005
4) Bertucci, F. et al. Gene expression profiling shows medullary breast cancer is a subgroup of basal breast cancers. Cancer Res. 66, 4636-44: 2006
5) Brenton JD, Carey LA, Ahmed AA, Caldas C. Molecular classification and molecular forecasting of breast cancer: ready for clinical application? J Clin Oncol 23: 7350-7360, 2005
6) Chang HY, Nuyten DSA, Sneddon JB, Hastie T, Tibshirani R, Sørlie T, Dai H, He YD, van't Veer LJ, Bartelink H, van de Rijn M, Brown PO, van de Vijver MJ. Robustness, scalability, and integration of a wound-response gene expression signature in predicting breast cancer survival. PNAS 102: 3738-3743, 2005
7) Chin Y, Janseens J, Vandepitte J, Vandenbrande J, Opdebeek L, Raus J. Phenotypic analysis of tumor-infiltrating lymphocytes from human breast cancer. Anticancer Res 12: 1463-1466, 1992
8) Coronella JA, Telleman P, Kingsbury GA, Truong TD, Hays S, Junghans RP. Evidence for an antigen-driven humoral immune response in medullary ductal breast cancer. Cancer Res 61: 7889-7899, 2001
9) Coronella JA, Spier C, Welch M, Trevor KT, Stopeck AT, Villar H, Hersh EM. Antigen-driven oligiclonal expansion of tumor-infiltrating B cells in infiltrating ductal carcinoma of the breast. J Immunol 169: 1829-1836,2002
10) Dai H, van't Veer L, Lamb J, He YD, Mao M, Fine BM, Bernards R, van de Vijver M, Deutsch P, Sachs A, Stoughton R, Friend S. A cell proliferation signature is a marker of extremely poor outcome in a subpopulation of breast cancer patients. Cancer Res 65: 4059-4066,2005
11) Di Paola M, Angelini L, Bertolotti A, Colizza S. Host resistance in relation to survival in breast cancer. Br Med J 4: 268-270, 1974
12) Foekens JA, Atkins D, Zhang Y, Sweep FCCJ, Harbeck N, Paradiso A, Cufer T, Sieuwerts AM, Talantov D, Span PN, Tjan-Heijnen VCG, Zito AF, Specht K, Hoefler H, Golouh R, Schittulli F, Schmitt, Beex LVAM, Klijn JGM, Wang Y. Multicenter Validation of a Gene Expression-Based Prognostic Signature in Lymph Node-Negative Primary Breast Cancer. J Clin Oncol 24:1665-71, 2006
13) Gaffey MJ, Frierson HF, Mills SE, Boyd JC, Zarbo RJ, Simpson JF, Gross LK, Weiss LM. Medullary carcinoma of the breast. Identification of lymphocyte subpopulations and their significance. Mod Pathol 6: 721-728, 1993
14) Gentili C, Sanfilippo O, Silvestrini R. Cell proliferation and its relationship to clinical features and relapse in breast cancers. Cancer 48: 974-979, 1981
15) Gruvberger S, Ringner M, Chen Y, Panavally S, Saal LH, Borg A, Fernö M, Peterson C, Meltzer PS. Estrogen receptor status in breast cancer is associated with remarkably distinct gene expression patterns. Cancer Res 61: 5979-5984, 2001
16) Gruvberger-Saal SK, Eden P, Ringner M, Baldetorp B, Chebil G, Borg A, Fernö M, Peterson C, Meltzer PS. Predicting continuous values of prognostic markers in breast cancer from microarray gene expression profiles. Mol Cancer Ther 3: 161-168, 2004
17) Hansen MH, Nielsen H, Ditzel HJ. The tumor-infiltrating B cell response in medullary breast cancer is oligoclonal and directed against the autoantigen actin exposed on the surface of apoptotic cancer cells. PNAS 98: 12659-12664,2001
18) Hansen MH, Nielsen HV, Ditzel HJ. Translocation of an intracellular antigen to the surface of medullary breast cancer cells early in apoptosis allows for an antigen-driven antibody response elicited by tumor-infiltrating B cells. J Immunol 169: 2701-2711, 2002
19) Kotlan B, Simsa P, Teillaud JL, Fridmann WH, Toth J, McKnight M, Glassy MC. Novel ganglioside antigen identified by B cells in human medullary breast carcinomas: the proof of principle concerning the tumor-infiltrating B lymphocytes. J Immunol 175: 2278-2285, 2005
20) Lucin K, Iternicka Z, Jonjic N. Prognostic significance of T-cell infiltrates, expression of beta 2-microglobulin and HLA-DR antigens in breast carcinoma. Pathol Res Pract 190: 1134-1140, 1994
21) Menard S, Tomasic G, Casalini P, Balsari A, Pilotti S, Cascinelli N, Salvadori B, Colnaghi MI, Rilke F. Lymphoid infiltration as a prognostic variable for early-onset breast carcinomas. Clin Cancer Res 3: 817-819, 1997
22) Nzula S, Going JJ, Stott DI. Antigen-driven clonal proliferation, somatic hypermutation, and selection of B lymphocytes infiltrating human ductal breast carcinomas. Cancer Res 63:3275-80,2003
23) Oh DS, Troester MA, Usary J, Hu Z, He X, Fan C, Wu J, Carey LA, Perou CM. Estrogen-regulated genes predict survival in hormone receptor-positive breast cancers. J Clin Oncol 24: 1656-1664, 2006
24) O'Sullivan C, Lewis CE. Tumour-associated leucocytes: friends or foes in breast carcinoma. J Pathol 172: 229-235, 1994
25) Osborne CK, Yochmowitz MG, Knight WA 3rd, McGuire WL. The value of estrogen and progesterone receptors in the treatment of breast cancer. Cancer 46: 2884-2888, 1980
26) Paik S, Shak S, Tang G, Kim C, Baker J, Cronin M, Baehner FL, Walker MG, Watson D, Park T, Hiller W, Fischer ER, Wickerham DL, Bryant J, Wolmark N. A multigene assay to predict recurrence of tamoxifen-treated, node-negative breast cancer. N Engl J Med 351: 2817-2826, 2004
27) Perou CM, Sorlie T, Eisen MB, van de Rijn M, Jeffrey SS, Rees CA, Pollack JR, Ross DT, Johnsen H, Akslen LA et al. Molecular portraits of human breast tumours. Nature 406: 747-752, 2000
28) Perrreard L, Fan C, Quackenbusch JF, Mullins M, Gauthier NP, Nelson E, Mone M, Hansen H, Buys SS, Rasmussen K, Ruiz Orrico A, Dreher D, Walters R, Parker J, Hu Z, He X, Palazzo JP, Olopade OI, Szabo A, Perou CM, Bernard PS. Classification and risk stratification of invasive breast carcinomas using a real-time quantitative RT-PCR assay. Breast Cancer Res 8: R23, 2006
29) Ridolfi R, Rosen P, Port A, Kinne D, Mike V. Medullary carcinoma of the breast. A clinicopathologic study with 10-year follow up. Cancer 40: 1365-1385, 1977
30) Roden JC, King BW, Trout D, Mortazavi A, Wold BJ, Hart CE. Mining gene expression data by interpreting principal components. BMC Bioinformatics 7: 194; 2006
31) Rouzier R, Perou CM, Symmans WF, Ibrahim N, Cristofanilli M, Anderson K, Hess KR, Stec J, Ayers M, Wagner P, Morandi P, Fan C, Rabiul I, Ross JS, Hortobagyi GN, Pusztai L. Breast cancer molecular subtypes respond differently to preoperative chemotherapy. Clin Cancer Res 11: 5678-5685, 2005
32) Shimokawara I, Imamura M, Yamanaka N, Ishii Y, Kikuchi K. Identification of lymphocyte subpopulations in human breast cancer tissue and its significance: an immunoperoxidase study with anti-human T- and B-cell sera. Cancer 49: 1456-1464; 1982
33) Sorlie T, Perou CM, Tibshirani, R, Aas T, Geisler S, Johnsen H, Hastie T, Eisen MB, van de Rijn M, Jeffrey SS, Thorsen T, Quist H, Matese JC, Brown PO, Botstein D, Lonning PE, Borresen-Dale AL. Gene expression patterns of breast carcinomas distinguish tumor subclasses with clinical implications. PNAS 98: 10869-10874, 2001
34) Sorlie T, Tibshirani R, Parker J, Hastie T, Marron JS, Nobel A, Deng S, Johnsen H, Pesich R, Geisler S, Demeter J, Perou CM, Lonning PE, Brown PO, Borresen-Dale AL, Botstein D. Repeated observation of breast tumor subtypes in independent gene expression data sets. PNAS 100: 8418-8423, 2003
35) Sotiriou C, Wirapati P, Loi S, Harris A, Fox S, Smeds J, Nordgren H, Farmer P, Praz V, Haibe-Kains B, Desmedt C, Larismont D, Cardoso F, Peterse H, Nuyten D, Buyse M, van de Vijver MJ, Bergh J, Piccart M, Delorenzi M. Gene expression profiling in breast cancer: Understanding the molecular basis of histologic grade to improve prognosis. JNCI 98: 262-272, 2006
36) Van de Vijver MJ, He YD, van't Veer LJ, Dai H, Hart AAM, Voskuil DW, Schreiber GJ, Peterse JL, Roberts C, Marton MJ, Parrish M, Atsma D, Witteveen A, Glas A, DeLahaye L, van der Velde T, Bartelink H, Rodenhuis S, Rutgers ET, Friend SH, Bernhards R. A gene-expression signature as a predictor of survival in breast cancer. N Engl J Med 347: 1999-2009, 2002
37) Van't Veer LJ, Dai H, van de Vijver MJ, He YD, Hart AAM, Mao M, Peterse HL, van der Kooy K, Marton MJ, Witteveen AT, Schreiber GJ, Kerkhoven RM, Roberts C, Linsley PS, Bernards R, Friend SH. Gene expression profiling predicts clinical outcome of breast cancer. Nature 415: 530-536, 2002
38) Wang Y, Klijn JGM, Zhang Y, Sieuwerts AM, Look MP, Yang F, Talantov D, Timmermans M, Meijer-van Gelder MF, Yu J, Jatkoe T, Berns EMJJ, Atkins D, Foekens JA. Gene-expression profiles to predict distant metastasis of lymph-node-negative primary breast cancer. Lancet 365: 671-679, 2005

The invention further includes the following embodiments.

### Items

1. Method for the prognosis of breast cancer in a breast cancer patient, said method comprising
   (a) determining the nodal status of said patient;
   (b) determining the expression level of at least one first marker gene in a tumor sample from said patient, said at least one first marker gene providing information on whether said tumor is fast proliferating or slow proliferating;
   (c) determining whether said tumor is a fast proliferating tumor or a slow proliferating tumor, by comparison of said expression level of said first marker gene with a predetermined first threshold level;
   (d) determining the expression level of at least one second marker gene in a tumor sample of said patient, wherein said second marker gene is specifically expressed in immune cells;
   wherein a favorable prognosis is given, if said nodal status is negative and said tumor is a slow proliferating tumor or a fast proliferating tumor and said expression level of said second marker gene is above a predetermined threshold level, and
   wherein an unfavorable prognosis is given if said nodal status is negative and said tumor is a fast proliferating tumor and said expression level of said second marker gene is below a predetermined threshold level.
2. Method of item 1, wherein said prognosis is based on the information that said nodal status is negative and that said tumor is a fast proliferating tumor and that said expression level of said second marker gene is below said predetermined threshold level.
3. Method of item 1 or 2, wherein said prognosis is an estimation of the likelihood of metastasis fee survival of said patient over a predetermined period of time.
4. Method of item 1 or 2, wherein said prognosis is an estimation of the likelihood of death of disease of said patient within a predetermined period of time.
5. Method of any one of the preceding items, wherein breast cancer patient is not treated with cancer chemotherapy.
6. Method of any one of the preceding items, wherein said first marker gene is selected from Table 1.
7. Method of any one of the preceding items, wherein said first marker gene is TOP2A or a gene co-regulated with TOP2A.
8. Method of any one of the preceding items, wherein said second marker gene is an immune globulin gene.
9. Method of any one of the preceding items, wherein said second marker gene shows specific expression in T-cells or specific expression in B-cells, or specific expression in natural killer cells.
10. Method of any one of the preceding items, wherein said second marker gene is selected from Table 2.
11. Method of any one of the preceding items, wherein said at least one second marker gene is IGHG3 or a gene co-regulated with Gene IGHG3.
12. Method of any of the preceding items, wherein the determination of expression levels is on a gene chip.
13. A system for performing prognosis of breast cancer in a breast cancer patient, said system comprising
   (a) means for storing information on the nodal status of said patient;
   (b) means for determining the expression level of at least one first marker gene;
   (c) means for comparing said expression level of said first marker gene with a predetermined first threshold value;
   (d) means for determining the expression level of at least one second marker gene; and
   (e) computing means programmed to give a favorable prognosis if said information on said nodal status indicates a negative nodal status and said comparison of said expression level of said first marker gene with said predetermined first threshold value indicates a fast proliferating tumor and said expression level of said second marker gene is above a predetermined second threshold level, and
   said computing means being programmed to give an unfavorable prognosis if said information on said nodal status indicates a negative nodal status and said comparison of said expression level of said first marker gene with said predetermined first threshold value indicates a fast proliferating tumor and said expression level of said second marker gene is below a predetermined second threshold level.
14. A system of item 13, wherein said prognosis is an estimation of the likelihood of metastasis free survival over a predetermined period of time.
15. A system of item 13 or 14, wherein said first marker gene is selected from Table 1.
16. A system of item 13 or 14, wherein said first marker gene is TOP2A or a gene co-regulated with TOP2A.
17. A system of any of items 13-16, wherein said second marker gene is an immune globulin gene.
18. A system of any of items 13-17, wherein said second marker gene expression in specific for T-cells or B-cells.
19. A system of any of items 13-18, wherein said second marker gene is selected from Table 2.
20. A system of any of items 13-19, wherein said second marker gene is IGHG3 or a gene co-regulated with IGHG3.
21. A system of any of items 13-20, wherein the determination of expression levels is on a gene chip.

## Claims

1. A method for the prognosis of breast cancer in a breast cancer patient, said method comprising:
(a) determining the nodal status of said patient;
(b) determining the expression level of at least one first marker gene in a tumor sample from said patient, said at least one first marker gene providing information on whether said tumor is fast proliferating or slow proliferating;
(c) determining whether said tumor is a fast proliferating tumor or a slow proliferating tumor, by comparison of said expression level of said first marker gene with a predetermined first threshold level;
(d) determining the expression level of at least one second marker gene in a tumor sample of said patient;
wherein the first and second genes are either BIRC5 and UBE2C, wherein a favorable prognosis is given, if said nodal status is negative and said tumor is a slow proliferating tumor or a fast proliferating tumor and said expression level of said second marker gene is above a predetermined threshold level, and wherein an unfavorable prognosis is given if said nodal status is negative and said tumor is a fast proliferating tumor and said expression level of said second marker gene is below a predetermined threshold level.

2. The method of claim 1, wherein said prognosis is an estimation of the likelihood of metastasis free survival of said patient over a predetermined period of time.

3. The method of claim 1, wherein said prognosis is an estimation of the likelihood of death of disease of said patient within a predetermined period of time.

4. The method of claim 1, wherein said patient has received endocrine therapy or is contemplated to receive endocrine therapy.

5. The method of any of the preceding claims, wherein the determination of expression levels is determined by at least one of a PCR-based method, a microarray-based method, and/or a hybridization based method.
